# EUROPEAN PATENT SPECIFICATION

(11) **EP 2 726 031 B1**
(45) Date of publication and mention of the grant of the patent: **12.08.2015**
(21) Application number: 12732781.5
(22) Date of filing: 29.06.2012
(51) Int. Cl.: A61F 5/443

(54) **AN OSTOMY APPLIANCE**
KÜNSTLICHER DARMAUSGANG
ACCESSOIRE POUR STOMIE

(30) Priority: 30.06.2011 DK 201170349
(43) Date of publication of application: 07.05.2014
(73) Proprietor: Coloplast A/S, 3050 Humlebæk (DK)
(72) Inventor: KLEIN, Charlotte, DK-2700 Broenshoej (DK); BUUS, Hasse, DK-3050 Humlebaek (DK)
(86) International application number: PCT/DK2012/050234
(87) International publication number: WO 2013/000480

(56) References cited:
- EP-A2- 0 726 068
- WO-A2-2004/087004

## Description

### FIELD OF THE I NVENTI ON

The present invention relates to an ostomy appliance comprising an adhesive wafer which comprises a first, a second and a third zone. The third zone defines at least a part of the outer rim of the adhesive wafer and the second zone encirculates the first zone.

### BACKGROUND OF THE I NVENTI ON

Ostomy appliances comprising a thin adhesive wafer are desirable as they are comfortable to wear. The reason is that the adhesive wafer is less noticed (or even not noticed at all) by the user, e.g. when the user bends the stomach region. However, such adhesive wafers suffer from the problem that they are difficult to remove from the skin of the user, as it is very difficult for the user to peel off the thin adhesive wafer.

In WO 2004/087004 is disclosed an ostomy wafer having three different annular zones. The zones may be provided with embossed pattern.

It is an object of one or more embodiments to overcome the abovementioned problem.

### DESCRIPTION OF THE I NVENTI ON

In a first aspect, the present invention relates to an ostomy appliance for attachment to a stoma, the ostomy appliance comprising:
an adhesive wafer having an outer rim, and an inner rim which is defined by a passage for receiving a stoma of a user, the adhesive wafer comprising a backing layer and a skin facing adhesive layer; and
a collecting bag which in use is secured to the adhesive wafer;
wherein the adhesive wafer in a first zone has a first set of properties and in a second zone has a second set of properties and in a third zone has a third set of properties;
wherein at least a part of the first zone is defined between the inner rim and the second zone; and

the third zone is defined between the outer rim and the second zone wherein the thickness of the third zone is at least 50% higher than the thickness of the second zone.

The collecting bag may be suitable for collecting waste material from the intestinal system of a human being. Accordingly, the ostomy appliance may be adapted for use in connection with colostomies, ileostomies or urostomies, both temporary and permanent types. However, the appliance may also be used for collecting fecal matter from the rectum of a person suffering from fecal incontinence without having undergone stoma surgery.

The passage defined in the adhesive wafer, may be circular, oval or polygonal in shape. It will be appreciated that in some embodiments, this passage is small enough to allow the user to customize the passage to the shape of the stoma of the user (i.e. the passage must be smaller than the stoma), while at the same time being large enough to allow a cutting means such as a scissor, to be inserted into the passage. Thus, in one embodiment, the largest dimension of the passage is below 50 mm, such as below 40 mm, such as below 30 mm, such as below 20 mm, such as below 15 mm, such as below 10 mm, such as below 5 mm

One more of the zones may comprise hydrocolloids. The hydrocolloids may be incorporated in the adhesive layer.

One or more of the zones may be substantially non-absorbent.

Preferred adhesives are soft adhesives, such as silicone or polyurethane adhesives. In one embodiment, the adhesive comprises a polyalkyleneoxide polymer and organosiloxane based cross-linked adhesive system. Other suitable adhesives may be styrene isoprene styrene block copolymer (SIS) or ethylene vinyl acetate (EVA) based adhesives.

In one embodiment, the backing layer comprises a polymer film, coating, laminate, textile or non-woven. The backing layer is preferably a highly flexible film, being strong enough for attachment of e.g. couplings and/or pouch and for removing the device in one piece, but soft enough to follow the movements of the body.

A preferred backing layer is a polyurethane film. Other suitable materials for the backing layer may be polyurethane/polyolefin blends or laminates.

Preferably, the backing layer has thermoplastic elements that enable welding of e.g. a pouch or coupling ring to the adhesive wafer. Preferred thickness of the backing layer is between 15-100 µm, 15-60 µm or 30-60 µm in order to maintain the softness of the adhesive wafer.

During use, the collecting bag is secured to the adhesive wafer. Thus in one embodiment, the ostomy appliance is a one-piece ostomy appliance in which the collecting bag is permanently secured to the adhesive wafer for example by welding or by adhesive means. In the latter embodiment, the collecting bag may be integrated with the wafer. The collecting bag may be secured to the adhesive wafer by means of an adhesive provided between the collecting bag and the adhesive wafer. The latter adhesive may be provided on the opposite side of the adhesive wafer than the skin facing adhesive layer. Alternatively, or as a supplement, the collecting bag may be coupled to the adhesive wafer by means of a mechanical coupling means.

In another embodiment, the collecting bag and the adhesive form a two-piece ostomy appliance which is delivered to the user in two pieces that must be connected to each other by the user. In the latter embodiment, the adhesive may be provided on mating surfaces of the collecting bag and the wafer. In one embodiment, the collecting bag is detachably attached to the adhesive wafer, i.e. in a way that the collecting bag may be detached from the adhesive wafer.

In one embodiment, the collecting bag is reattachably attached to the adhesive wafer. By reattachably attached shall be understood that the collecting bag is attached or attachable to the adhesive wafer in a way that it may subsequently be detached and reattached to the adhesive wafer.

In one embodiment, the shape of the adhesive wafer is round such as circular or oval. The skin facing adhesive layer may be covered by a release liner. The release liner may be siliconised or otherwise provided with a non-stick surface on the side which faces the skin facing adhesive layer. This release must be removed in order for the user to adhere the skin facing adhesive layer to the skin of the user.

The inner and/or the outer rim of the wafer may be bevelled in order to provide a smooth transition to the skin. Usually, bevelled edges have a slope defining an angle with the skin-facing surface of 45 degrees or less and may further terminate in a thin flange.

However, such bevelled edge may be difficult to release and grip with the fingers when detaching the wafer. In order to facilitate easy gripping and peeling of the edge, the third zone may be bevelled in a steep angle, being higher than 45 degrees, such as higher than 50 degrees, such as higher than 60 degrees, such as higher than 70 degrees such as higher than 80 degrees such as higher than 90 degrees, to the skin facing surface, or the edge may not be bevelled at all at the third zone, but terminate in a cut substantially perpendicular to the skin-facing surface. The angle is measured as the angle between the skin-facing surface and the bevelled edge.

The first zone defines an inner rim of the adhesive wafer. This inner rim is defined by the passage of the adhesive wafer. In use, waste exiting the stoma flows into the collecting bag through the passage in the ostomy appliance.

At least a part of the first zone is defined between the at least a part of inner rim and at least a part of the second zone. In other words, at least a part of the first zone is encirculated by the second zone. In one embodiment, at least 30 percent of the first zone is encirculated by the second zone, such as at least 50 percent, such as at least 60 percent, such as at least 70 percent, such as at least 80 percent, such as at least 90 percent, such as at least 100 percent. In the latter case, the first zone is fully encirculated by the second zone.

By providing a third zone at the rim, which have properties which are different than those of the second zone, it may be possible to achieve the advantages of a thin adhesive wafer (e.g. the second zone may be provided as a thin adhesive wafer), while at the same time increasing the ability to peel the adhesive wafer by providing the third zone, which may comprise a material which is easier to peel, e.g. by providing the third zone as a relatively thick adhesive wafer.

The thickness of the third zone is at least 20% higher than the thickness of the second zone, thereby facilitating easy tactile identification of place for initiating detachment as well as easing grip and detachment. The thickness of the third zone may be 25%, such as 30%, such as 40%, such as 50%, such as 60%, such as 70%, such as 80%, such as 90%, such as 100%, such as 125% such as 150%, such as 200%, such as 250%, such as 300% higher than the second zone.

The thickness of the second zone may be 100-1000 µm, such as 300-800 µm, such as 300-600 µm.

The thickness of the third zone may be 500-2500 µm, such as 1000-1500 µm, such as 1000-1200 µm.

At least a part of the transition line between the second and the third zone may be provided with a notch. The notch is in the form of a depression, i.e. an area where the wafer is thinner than the neighboring second and third zones. The notch may comprise the entire transition line. Preferably, the notch is substantially parallel to the rim of the wafer. When the third portion is loosened from the skin, it may tip over in a rolling motion with the notch working as a hinge, and thereby ease detachment of the wafer.

In one embodiment, the radial dimension of first zone from the inner rim to the transition between the first and the second zone constitutes approximately 10 percent of the radial dimension of the adhesive wafer from the inner rim to the outer rim, such as 20 percent, such as 30 percent, such as 40 percent, such as 10-50 percent.

In one embodiment, the radial dimension of the second zone from the transition with the first zone to the outer rim /or the transition with the third zone constitutes approximately 10 percent of the radial dimension of the adhesive wafer from the inner rim to the outer rim, such as 20 percent, such as 30 percent, such as 40 percent, such as 10-50 percent.

In one embodiment, the radial dimension of the third zone from the outer rim to the transition with the first or the second zone constitutes approximately 10 percent of the radial dimension of the adhesive wafer from the inner rim to the outer rim, such as 20 percent, such as 30 percent, such as 40 percent, such as 10-50 percent.

The third zone defines at least a part of the outer rim of the adhesive wafer. In one embodiment, the outer rim is only defined by the third zone. In the latter embodiment, the third zone fully encirculates the second zone.

In one embodiment, a first part of the outer rim of the adhesive wafer is defined by the second zone and a second part of the rim is defined by the third zone. In one embodiment, the outer rim is only defined by the first and the second part, while in other embodiments, further parts (e.g. a third part) may be defined by the outer rim of the adhesive wafer.

In one embodiment, the outer and/or the inner rim may have a decreasing thickness in the direction of the rim. Accordingly, the rim may be said to form an inclined surface or be bevelled.

In one embodiment, a circumferential length of the first part of the outer rim is at least three times the circumferential length of the second part of the rim. Alternatively, the circumferential length of the first part of the outer rim is at least one and a half times the circumferential length of the second part of the rim, such as two times, such as four times, such as at least five times.

In one embodiment, two of the first, second and third zones are identical with regard to one or more properties. As an example all the properties of said two zones are identical, e.g. the two zones may be made from the same material. In the latter embodiment, the thickness, the tear strength etc. may be identical in the two zones. Alternatively, only some of the properties are identical whereby other properties of the two zones are non-identical.

In one embodiment, the first and third set of properties are identical, whereby the properties of the first and the third zones are identical. As mentioned previously, a thick third zone may increase the peelability, meaning that it makes it easier to loosen the edge portion, typically with the finger tips, in order to get a grip of the edge portion for detachment of the adhesive wafer. Accordingly, a thick third zone may enable the user to disconnect the adhesive wafer from the skin of the user in the area of the third zone and to peel the remaining part of the adhesive wafer from the skin of the user. Thus, even in cases where the second zone is made from a thin material, the user may be able to remove the adhesive wafer. In the latter embodiment, the first and the third zone may be made from a thick material.

Alternatively, the first and the second set of properties may be identical, or the second and the third set of properties may be identical.

The first, second and the third zones are defined by the adhesive wafer. In one embodiment, one or more of the first, the second and the third zones are defined by the skin facing adhesive layer. Alternatively, or as a supplement, one or more of the first, second and third zones may be defined by the backing layer.

In one embodiment, one or more of the first, second and third set of properties comprises one or more of: a thickness of the first and/or second and/or third zone, or a tear strength of the first and/or the second and/or the third zone, or a stiffness of the first and/or second and/or the third zone, or a flexibility of the first and/or the second and/or the third zone.

### BRIEF DESCRIPTION OF THE FIGURES

The invention will now be described with reference to the figures in which:
Fig. 1 discloses a front elevational view of an embodiment of the adhesive wafer,
Fig. 2 discloses a front elevational view of another embodiment of the adhesive wafer,
Fig. 3 discloses a cross section of an ostomy appliance comprising an adhesive wafer according to the first embodiment and
Fig. 4 discloses an enlarged view of the section marked "A" in Fig. 3.

### DETAILED DESCRIPTION OF THE FIGURES

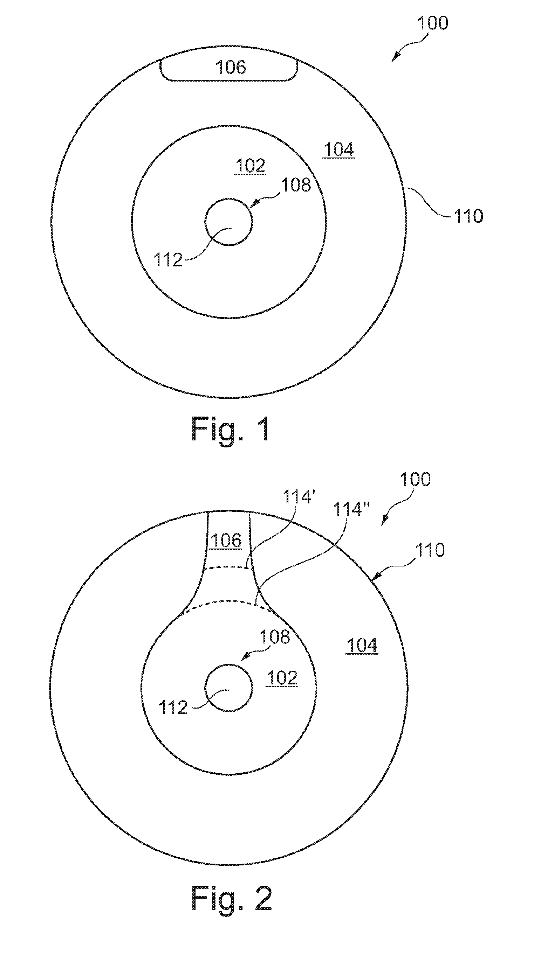

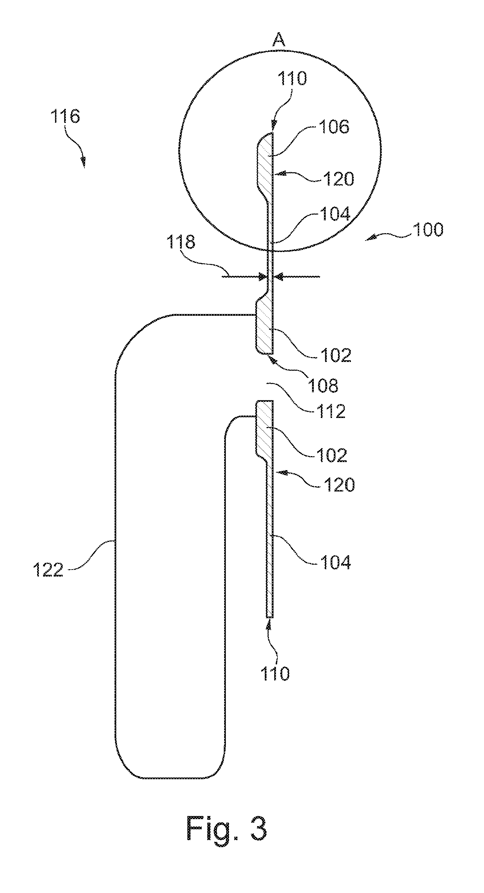

Fig. 1 discloses an ostomy appliance (shown in Fig. 3) comprising an adhesive wafer 100 comprising a first zone 102, a second zone 104 and a third zone 106. The adhesive wafer 100 defines an inner rim 108 and an outer rim 110, the inner rim 108 is defined in the transition between the adhesive wafer 100 and the passage 112. The first zone 102 defines the inner rim 108 and is encirculated by the second zone 104. In the embodiment of Fig. 1 the second zone 104 fully encirculates the first zone 102, however it will be appreciated that in other examples, only a part of the first zone 102 is encirculated by the second zone 104. As an example the latter is the case in Fig. 2.

A first part of the outer rim 110 of the adhesive wafer 100 is defined by the second zone 104 while the remaining part of the outer rim 110 is defined by the third zone 106. Accordingly in Fig. 1, the third zone 106 is defined by a part of the outer rim 110 and a part of the second zone 104.

In one embodiment, the first zone 102 is provided in the form of a material which causes the adhesive wafer 100 to be leak proof in the area of the passage. Moreover, the first zone 102 may have good adherence to the skin in a way that likelihood of unintended detachment from the skin of the first zone 102 is prevented or reduced. The first zone 102 may comprise a pressure sensitive adhesive such as a hydrocolloid adhesive. However, as the latter kind of adhesives are often seen as being relatively stiff, a less stiff (and thus more flexible) second zone 104 is provided, which may be provided in the form of a thin and flexible material. However, such thin and flexible materials may be difficult to peel off, and thus the third zone 106, which may be made from a thicker and/or stiffer material is provided. As an example, the third zone 106 and the first zone 102 may be made from the same material, e.g. a hydrocolloid adhesive may be provided in the area of the first zone 102 and the third zone 106.

In the embodiment of Fig. 2, the first zone 102 and the third zone 106 form a unitary zone. In one embodiment, the two zones form a monolithic zone without seams. In another embodiment, the two zones are separated by a seam 114' or 114". The unitary zone may define a center part (corresponding to the first zone 102) and a radially extending part (corresponding to the third zone 106). The width of the radially extending part (the third zone 106) decreases in the direction towards the outer rim 110 of the adhesive wafer 100.

Fig. 3 discloses a cross-sectional view of an ostomy appliance 116 comprising an adhesive wafer 100 according to the first embodiment, cf. Fig. 1. It will be appreciated that the first zone 102 and the third zone 106 are thicker than the second zone 104 when seen in the direction of the arrows 118. By providing the relatively thick third zone 106, it may be possible for the user to peel off the entire adhesive wafer 100, as the third zone 106 has a thickness that allows for detaching the adhesive wafer 100 in the area of the third zone 106.

The reason for this is that the thickness enables the user to force a nail in-between the skin of the user and the skin facing adhesive layer 120. Once a part of the skin facing adhesive layer 120 has been loosened by the user, the user may pull the adhesive wafer 100 off the skin by grapping onto the adhesive wafer 100 by means of the index finger and the thumb.

In the embodiment of Fig. 3, a single piece ostomy appliance 116 is illustrated in which the collecting bag 122 is secured directly to the adhesive wafer 100, which in use is adhered directly to the skin of the user. However, in other embodiments, the user must secure (e.g. by means of an adhesive) the collecting bag 122 to the adhesive wafer 100. In the latter embodiment, the adhesive wafer 100 may be reused once a full collecting bag has been removed.

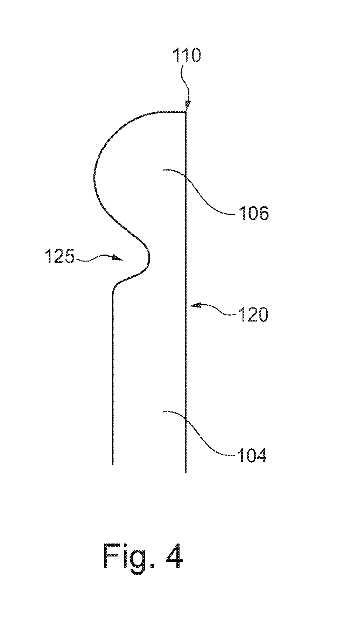

Figure 4 discloses a close up picture of the encircled section marked "A" of Figure 1. The third zone 106 extends from the outer rim 110 of the wafer to the second zone 104 and is in the form of a raised portion of increased thickness. The part of the third zone 106 facing the rim 110 of the wafer terminates in a steep angle in order to make the distinction between the skin surface and the wafer more distinct and easier to grip with the fingers for detachment of the wafer. In order to further ease the detachment, the wafer may be provided with a notch 125 between the third zone 106 and the second zone 104. The notch 125 facilitates rolling of the third zone 106, thereby making it easier to release the third zone 106 from the skin surface and get a good grip around it.

## Claims

1. An ostomy appliance for attachment to a stoma, the ostomy appliance comprising:
an adhesive wafer (1) having an outer rim (110), and an inner rim (108) which is defined by a passage (112) for receiving a stoma of a user, the adhesive wafer comprising a backing layer and a skin facing adhesive layer; and
a collecting bag which in use is secured to the adhesive wafer;
wherein the adhesive wafer (1) in a first zone (102) has a first set of properties and in a second zone (104) has a second set of properties and in a third zone (106) has a third set of properties;
wherein at least a part of the first zone (102) is defined between the inner rim (108) and the second zone (104); and
the third zone (106) is defined between the outer rim (110) and the second zone (104),
**characterized in that** the thickness of the third zone (106) is at least 50% higher than the thickness of the second zone (104).

2. An ostomy appliance according to claim 1, wherein the first zone (102) is encirculated by the second zone (104).

3. An ostomy appliance according to any of the preceding claims, wherein a first part of the outer rim of the adhesive wafer is defined by the second zone (104) and a second part of the outer rim (110) is defined by the third zone (106).

4. An ostomy appliance according to claim 3, wherein a circumferential length of the first part of the outer rim (110) is at least three times the circumferential length of second part of the outer rim (110).

5. An ostomy appliance according to any of the preceding claims, wherein the first and third set of properties are identical.

6. An ostomy appliance according to any of the preceding claims, wherein the first and the second set of properties are identical.

7. An ostomy appliance according to any of the preceding claims, wherein one or more of the first (102), the second (104) and the third zones (106) are defined by the skin facing adhesive layer.

8. An ostomy appliance according to any of the preceding claims, wherein one or more of the first (102), second (104) and third zones (106) are defined by the backing layer.

9. An ostomy appliance according to any of the preceding claims, wherein one or more of the first, second and third set of properties comprise one or more of: a thickness of the first (102) and/or second (104) and/or third zone (106), or a tear strength of the first (102) and/or the second (104) and/or the third zone (106), or a stiffness of the first (102) and/or second (104) and/or third zone (106).

10. An ostomy appliance according to any of the preceding claims, wherein at least a part of a transition line between the second (104) and the third zone (106) is provided with a notch.

## Patentansprüche

1. Ostomievorrichtung zur Anbringung an einem Stoma, wobei die Ostomievorrichtung Folgendes umfasst:
eine Klebescheibe (1) mit einem äußeren Rand (110) und einem inneren Rand (108), der durch einen Durchgang (112) zur Aufnahme eines Stomas eines Benutzers definiert ist, wobei die Klebescheibe eine Trägerschicht und eine zur Haut weisende Klebeschicht umfasst, und
einen Sammelbeutel, der im Gebrauch an der Klebescheibe befestigt ist,
wobei die Klebescheibe (1) in einer ersten Zone (102) eine erste Menge von Eigenschaften und in einer zweiten Zone (104) eine zweite Menge von Eigenschaften und in einer dritten Zone (106) eine dritte Menge von Eigenschaften hat,
wobei mindestens ein Teil der ersten Zone (102) zwischen dem inneren Rand (108) und der zweiten Zone (104) definiert ist und
die dritte Zone (106) zwischen dem äußeren Rand (110) und der zweiten Zone (104) definiert ist,
**dadurch gekennzeichnet, dass** die Dicke der dritten Zone (106) mindestens 50% höher als die Dicke der zweiten Zone (104) ist.

2. Ostomievorrichtung nach Anspruch 1, wobei die erste Zone (102) durch die zweite Zone (104) eingekreist ist.

3. Ostomievorrichtung nach einem der vorhergehenden Ansprüche, wobei ein erster Teil des äußeren Rands der Klebescheibe durch die zweite Zone (104) definiert ist und ein zweiter Teil des äußeren Rands (110) durch die dritte Zone (106) definiert ist.

4. Ostomievorrichtung nach Anspruch 3, wobei eine Umfangslänge des ersten Teils des äußeren Rands (110) mindestens das Dreifache der Umfangslänge des zweiten Teils des äußeren Rands (110) beträgt.

5. Ostomievorrichtung nach einem der vorhergehenden Ansprüche, wobei die erste und die dritte Menge von Eigenschaften identisch sind.

6. Ostomievorrichtung nach einem der vorhergehenden Ansprüche, wobei die erste und die zweite Menge von Eigenschaften identisch sind.

7. Ostomievorrichtung nach einem der vorhergehenden Ansprüche, wobei eine oder mehrere der ersten (102), der zweiten (104) und der dritten Zone (106) durch die zur Haut weisende Klebeschicht definiert sind.

8. Ostomievorrichtung nach einem der vorhergehenden Ansprüche, wobei eine oder mehrere der ersten (102), der zweiten (104) und der dritten Zone (106) durch die Trägerschicht definiert sind.

9. Ostomievorrichtung nach einem der vorhergehenden Ansprüche, wobei eine oder mehrere der ersten, zweiten und dritten Menge von Eigenschaften eine oder mehrere der Folgenden umfassen: eine Dicke der ersten (102) und/oder zweiten (104) und/oder dritten Zone (106) oder eine Reißfestigkeit der ersten (102) und/oder der zweiten (104) und/oder der dritten Zone (106) oder eine Steifheit der ersten (102) und/oder zweiten (104) und/oder dritten Zone (106).

10. Ostomievorrichtung nach einem der vorhergehenden Ansprüche, wobei mindestens ein Teil einer Übergangslinie zwischen der zweiten (104) und der dritten Zone (106) mit einer Kerbe versehen ist.

## Revendications

1. Appareillage pour stomie destiné à être attaché à une stomie, l'appareillage pour stomie comprenant :
une plaquette adhésive (1) comportant un bord extérieur (110) et un bord intérieur (108) qui est défini par un passage (112) destiné à recevoir une stomie d'un utilisateur, la plaquette adhésive comprenant une couche de support et une couche adhésive orientée vers la peau ; et
une poche de collecte qui est, lors de l'utilisation, fixée à la plaquette adhésive ;
la plaquette adhésive (1) possédant, dans une première région (102), un premier ensemble de propriétés et, dans une deuxième région (104), un deuxième ensemble de propriétés et, dans une troisième région (106), un troisième ensemble de propriétés ;
au moins une partie de la première région (102) étant définie entre le bord intérieur (108) et la deuxième région (104) ; et
la troisième région (106) étant définie entre le bord extérieur (110) et la deuxième région (104),
**caractérisé en ce que** l'épaisseur de la troisième région (106) est supérieure d'au moins 50 % à l'épaisseur de la deuxième région (104).

2. Appareillage pour stomie selon la revendication 1, dans lequel la première région (102) est entourée par la deuxième région (104).

3. Appareillage pour stomie selon l'une quelconque des revendications précédentes, dans lequel une première partie du bord extérieur de la plaquette adhésive est définie par la deuxième région (104) et une deuxième partie du bord extérieur (110) est définie par la troisième région (106).

4. Appareillage pour stomie selon la revendication 3, dans lequel une longueur circonférentielle de la première partie du bord extérieur (110) est égale à au moins trois fois la longueur circonférentielle de la deuxième partie du bord extérieur (110).

5. Appareillage pour stomie selon l'une quelconque des revendications précédentes, dans lequel les premier et troisième ensembles de propriétés sont identiques.

6. Appareillage pour stomie selon l'une quelconque des revendications précédentes, dans lequel les premier et deuxième ensembles de propriétés sont identiques.

7. Appareillage pour stomie selon l'une quelconque des revendications précédentes, dans lequel une ou plusieurs régions parmi les première (102), deuxième (104) et troisième (106) régions sont définies par la couche adhésive orientée vers la peau.

8. Appareillage pour stomie selon l'une quelconque des revendications précédentes, dans lequel une ou plusieurs régions parmi les première (102), deuxième (104) et troisième (106) régions sont définies par la couche de support.

9. Appareillage pour stomie selon l'une quelconque des revendications précédentes, dans lequel un ou plusieurs ensembles de propriétés parmi les premier, deuxième et troisième ensembles de propriétés comprennent une ou plusieurs des propriétés suivantes : une épaisseur de la première (102) et/ou de la deuxième (104) et/ou de la troisième (106) région, ou une résistance à la déchirure de la première (102) et/ou de la deuxième (104) et/ou de la troisième (106) région, ou une rigidité la première (102) et/ou de la deuxième (104) et/ou de la troisième (106) région.

10. Appareillage pour stomie selon l'une quelconque des revendications précédentes, dans lequel au moins une partie d'une ligne de transition entre les deuxième (104) et troisième (106) régions comporte une échancrure.
